(19) Europäisches Patentamt
European Patent Office
Office européen des brevets

(11) **EP 2 321 613 B1**

(12) **EUROPEAN PATENT SPECIFICATION**

(45) Date of publication and mention
of the grant of the patent:
**04.09.2019 Bulletin 2019/36**

(21) Application number: **08757269.9**

(22) Date of filing: **18.06.2008**

(51) Int Cl.:
*G01B 7/26* (2006.01)          *A61B 5/103* (2006.01)
*G01N 27/00* (2006.01)          *H01P 3/00* (2006.01)
*A61B 18/18* (2006.01)          *G01R 27/26* (2006.01)
*A61B 5/00* (2006.01)          *A61B 5/053* (2006.01)
*A61K 49/00* (2006.01)

(86) International application number:
**PCT/CH2008/000276**

(87) International publication number:
**WO 2009/152625 (23.12.2009 Gazette 2009/52)**

(54) **METHOD FOR CHARACTERIZING THE EFFECT OF A SKIN TREATMENT AGENT ON SKIN**

VERFAHREN ZUR CHARAKTERISIERUNG DER AUSWIRKUNG EINES HAUTBEHANDLUNGSMITTELS AUF DIE HAUT

MÉTHODE DE CARACTÉRISATION DE L'EFFET D'UN AGENT DE TRAITEMENT CUTANÉ SUR LA PEAU

(84) Designated Contracting States:
**AT BE BG CH CY CZ DE DK EE ES FI FR GB GR HR HU IE IS IT LI LT LU LV MC MT NL NO PL PT RO SE SI SK TR**

(43) Date of publication of application:
**18.05.2011 Bulletin 2011/20**

(73) Proprietor: **Biovotion AG**
**8008 Zürich (CH)**

(72) Inventors:
• **MEGEJ, Alexander**
**CH-8044 Zürich (CH)**
• **CADUFF, Andreas**
**CH-8005 Zürich (CH)**
• **TALARY, Mark**
**CH-8041 Zürich (CH)**

(74) Representative: **Sutter, Kurt**
**E. Blum & Co. AG**
**Vorderberg 11**
**8044 Zürich (CH)**

(56) References cited:
WO-A-2004/049937          WO-A-2005/120332
WO-A-2007/053963          WO-A-2008/154753
US-A1- 2007 190 006

• **LACKERMEIER A H ET AL: "IN VIVO AC IMPEDANCE SPECTROSCOPY OF HUMAN SKIN THEORY AND PROBLEMS IN MONITORING OF PASSIVE PERCUTANEOUS DRUG DELIVERY" ANNALS OF THE NEW YORK ACADEMY OF SCIENCES, NEW YORK ACADEMY OF SCIENCES, NEW YORK, NY, US, vol. 873, 20 April 1999 (1999-04-20), pages 197-213, XP008029774 ISSN: 0077-8923**
• **YAMAMOTO Y ET AL: "MEASUREMENT PRINCIPLE FOR EVALUATING THE PERFORMANCE OF DRUGS AND COSMETICS BY SKIN IMPEDANCE" MEDICAL AND BIOLOGICAL ENGINEERING AND COMPUTING, SPRINGER, HEILDELBERG, DE, vol. 16, no. 6, 1 November 1978 (1978-11-01), pages 623-632, XP002332620 ISSN: 0140-0118**

EP 2 321 613 B1

**Description**

Technical Field

The present description relates to a method and a device for characterizing an effect that a skin treatment agent, such as a soap or creme, has on a region of skin, in particular how the agent affects the humidity and physical properties in the various skin layers.

Background Art

The characterization of skin treatment agents is of particular interest in cosmetics, cleansing and pharmaceutical fields. In order to assess the effect of such agents on the body, a good knowledge of the response of the body to the agent is required. Of particular interest is the response of the skin to the agent, and most notably the response of skin humidity.

Therefore, a number of methods and devices have been proposed for quantitatively characterizing skin treatment agents.

US 6 442 408 describes, for example, an optical method using NIR light. Other devices, such as described in US 6 966 877, use humidity sensors for monitoring transdermal water loss.

Yet other devices, such as e.g. described in Patent Abstracts of Japan, JP 56118654, use electrical measurements for determining skin humidity.

A tissue mapping system having an array of electrodes has been described in WO 2004/049937. WO 2008/154753 describes a method for measuring the response of the tissue of a human or animal body to an electromagnetic field, and is prior art under Art. 54(3) EPC.

Disclosure of the Invention

It is a general object of the invention to provide a method of this type that provides further information on the effect of a non-therapeutic agent to the skin.

This object is achieved by the method of claim 1. Accordingly, the method comprises the following steps:

(a) The non-therapeutic skin treatment agent is applied to the skin region.

(b) A measuring device is applied to the skin region. The measuring device has several sets of electrodes, with each set comprising at least two electrodes. The electrodes of each set have a mutual distance $W_i$ from each other, and there are $N > 1$ sets having differing distances $W_i$.

(c) By means of the electrodes, at least $N$ electrical fields are generated within the skin region, wherein these fields have differing penetration depths into the skin region.

(d) $N$ "measured parameters" $m_i$ are determined, wherein each measured parameter $m_i$ depends on an effective permittivity as seen by a different one of the $N$ electrical fields. Hence, the measured parameters are descriptive of different penetration depths of the layers of the skin.

(e) At least one "characterizing parameter" is derived from the measured parameters $m_i$. The characterizing parameter is descriptive of the electrical permittivity of the skin region for a given depth.

At least one electrode set has an electrode distance of 100 $\mu$m or less in order to obtain a measurement specific for the stratum corneum. Similarly, at least one other electrode set has a gap width $W$ of at least 0.1 mm in order to obtain a measurement indicative of epidermis or dermis properties.

The characterizing parameters can e.g. be dependent on the permittivity of a single skin layer only, i.e. it is independent of the permittivities of layers. Alternatively, it may be an average permittivity up to a certain depth, in which case it is reasonable to compare several such characterizing parameters.

The invention is based on the understanding that, for a more complete assessment of the effects of a skin treatment agent, it is useful not only to know about the moisture of a topmost part of the skin, as it is measured by conventional devices, but to know about the moisture at one or more well defined depths below the skin surface.

In general, an advantageous embodiment of the invention includes the calculation of several characterizing parameters for differing layers of the skin. This allows to assess the amount of water at differing depths, thereby gaining more detailed knowledge of the moisture structure of the skin.

The term "skin treatment agent" is to be understood in a broad manner and includes any substances that can be expected to come into contact with the skin and who may have an effect on the dielectric properties of the skin. Examples: soaps and detergents, paints, substances used for make-ups, cosmetic crèmes, gels, emulsions and lotions, deodorants, oils, hair sprays, shampoos, sun blockers or therapeutic agents.

The method of the present invention is suited for characterizing non-therapeutic agents, such as cosmetic crèmes, gels, soaps, body paints, hydration agents, etc (see list above). According to an illustrative embodiment of the description which does not form part of the present invention, it is also suited for characterizing therapeutic agents, such as medicinal crèmes.

The invention relates to a method for characterizing non-therapeutic skin treatment agents.

It can e.g. be used for understanding and improving such agents or for detecting incompatibilities.

The present description also relates to a device for being used in a method for characterizing skin treatment agents. This device comprises a plurality of coplanar waveguides having differing gap widths (electrode distances) for generating the fields as used in the method described above.

The device is particularly suited for characterizing effects of the non-therapeutic agent on the skin of humans and, in general, mammals.

Brief Description of the Drawings

The invention will be better understood and objects other than those set forth above will become apparent when consideration is given to the following detailed description thereof. Such description makes reference to the annexed drawings, wherein:

Fig. 1 is a sectional view of a coplanar waveguide,
Fig. 2 is a sectional view of a conductor-backed coplanar waveguide,
Fig. 3 shows a graphical representation of the measurement system based on the CBCPW,
Fig. 4 is a block diagram of a device for measuring a parameter,
Fig. 5 is a device carrying two CPWs as seen from the side facing the sample,
Fig. 6 is an alternative CPW geometry,
Fig. 7 shows a device with a two-layer skin region above it,
Fig. 8 shows a first test measurement using a first agent,
Fig. 9 shows a second test measurement using a second agent.

Modes for Carrying Out the Invention

**1. Introduction**

***1.1. Human Skin Structure***

The skin can be basically divided into two major parts. The *epidermis* - the outer skin - comprises the stratum corneum, stratum granulosum and stratum spinosum and forms a waterproof, protective wrap over the human body surface. It does not contain any blood vessels and is nourished by diffusion from the dermis, the underlying skin layer. The underlying *dermis* is the layer of the skin that consists of connective tissue and cushions the body from stress and strain. The dermis is tightly connected to the epidermis by the basement membrane. It also harbors many nerve endings that provide the sense of touch and heat. It contains the hair follicles, sweat glands, sebaceous glands, apocrine glands and blood vessels. The blood vessels in the dermis provide nourishment and waste removal to and from its own cells as well as the stratum basale of the epidermis. A model of the human skin for electromagnetic simulations is described in detail in the next section.

1.1.1. Skin Structure and Its EM Modelling

Table 1 summarizes a dielectric model as an example of the skin at the upper arm. The parameters of this model are given for static DC conditions only, which does not correspond to the dielectric behaviour in reality; but they provide a first estimate for an initial design. It has to be noted that the thickness of single layers strongly depends on the observation site of the human body.

**TABLE 1:** Dielectric model of human skin (static DC conditions)

| Layer | Thickness, $t/m$ | Rel. Permittivity, $\varepsilon_r$ | Conductivity, $\sigma$ / S/m |
|---|---|---|---|
| Sebum | 4E-6 | 25 | 1E-3 |
| Stratum | 15-100E-6 | 10 (higher for humid) | 1E-4 to 1E-5 |
| Epidermis | 100-200E-6 | 20 | 0.025 |

(continued)

| Layer | Thickness, $t/m$ | Rel. Permittivity, $\varepsilon_r$ | Conductivity, $\sigma$ / S/m |
|---|---|---|---|
| Dermis | 1E-3 | 110 | 0.2 |
| Fat | 1.2E-3 | 20 | 2E-4 |
| Muscle | 20E-3 | 80 | 0.7 |

The *sebum* layer of the skin describes the substance secreted by the sebaceous glands. It mainly consists of fat and the debris of dead fat-producing cells. Sebum protects and waterproofs hair and skin, and keeps them from becoming dry, brittle, and cracked. For electro-magnetic simulations, it is modelled to have a permittivity of some 25.

Strictly speaking, the *stratum corneum* is a part of the epidermis layer of the skin. It has, however, slightly different properties from the electro-magnetic point of view. Due to the different conductivity, it may be modeled as an additional layer. In physiological terms, stratum corneum is the outermost layer of the *epidermis.* It is mainly composed of dead cells. As these dead cells slough off, they are continuously replaced by new cells from the underlying layers. Cells of the stratum corneum contain keratin, a protein that helps keep the skin hydrated by preventing the water evaporation. In addition, these cells can also absorb water, further aiding in hydration. The permittivity and conductivity of this layer is assumed to be variable and dependent on the fact whether the skin is wet or not.

Due to the high concentration of protein fibres, the *dermis* layer has got a very high permittivity of ~110, while the presence of the blood and the interstitial fluid increases its conductivity in comparison to the surrounding tissues.

The deeper layers, which have to be considered for the development of sensors having large electrode gaps, are the fat and the muscle compartments. The fat is the main component of the subcutaneous tissue (also called hypodermis). The muscle tissue is set to be the boundary for the EM model as it is assumed to have relatively high values of thickness (20mm) and conductivity (0.7 S/m).

### 1.2. Layer model

In an advantageous embodiment of the present invention, it is assumed that a skin region to be tested is composed of a layered structure having a plurality of homogeneous layers $i$ with $i = 1 \ldots N$ and $N > 1$, with layer $N$ being the topmost layer, i.e. the layer comprising one or more of the outermost layers of the skin. The individual layers have thicknesses $h_i$. $h_1$ is assumed to be infinite. The other thicknesses $h_2 \ldots h_N$ may be equal or not equal to each other.

The linear response of each layer to an applied electric field is described by its permittivity $\varepsilon_i$. In general, the permittivity $\varepsilon_i$ is a complex number having a real part $\varepsilon'_i$ and an imaginary part $\varepsilon''_i$. In a simple model, the imaginary part $\varepsilon''_i$ can be assumed to be zero (lossless case, zero conductivity), while a refined model can take non-zero imaginary parts $\varepsilon''_i$ into account. Methods for calculating how an electrical field is affected by such multi-layer systems, and in particular what effective permittivity $\varepsilon_{eff}$ the field experiences, are known to the skilled person.

## 2. Sensor Implementations

### 2.1. Introduction

The present invention relies on using a sensor device that is able to perform a depth-resolved measurement on a skin region having a structure as described under section 1.2. In an advantageous embodiment, this sensor comprises one or more coplanar waveguides as described under section 2.2.

It must be noted, though, that the present invention can also be carried out with other electrode geometries, such as with the device disclosed in WO 2005/120332, the disclosure of which, in particular regarding the electrode geometries and evaluation circuitry, is incorporated by reference herein.

In general, such a sensor has a plurality of electrodes that can be used to form at least $N$ sets of electrodes. In the example of the coplanar waveguides as described below, each coplanar waveguide forms one such electrode set. The distance $W_i$ between the electrodes of each set differ from each other.

The sensor device is applied to the skin region under test with the electrodes being close to the topmost layer of the skin. The electrode sets are then used to generate at least $N$ electrical fields within the skin region, wherein the electrical fields have differing penetration depths into the skin region. The different electrical fields can be applied sequentially, or (if a cross-talk between electrodes can be neglected or is compensated for) the fields can be applied concurrently.

The characteristics of the fields will be a function of the differing effective permittivities $\varepsilon_{eff}$, depending on how far the fields penetration into the skin/tissue. These effective permittivities describe the linear response (polarization) of the tissue to the fields.

For each field or used electrode set, a "measured parameter" $m_i$ is measured. This parameter may e.g. be the electrical impedance $Z$ or capacitance $C$ of the corresponding set of electrodes, or a phase shift or damping coefficient for a signal passing through said set of electrodes, and it will depend on the effective permittivity experienced by said set of electrodes.

Using e.g. the techniques as described below, the measured parameters $m_i$ can be converted, by means of suitable calculations, into one or more "characterizing parameters" $p_i$, in particular with each characterizing parameter $p_i$ e.g. depending on the permittivity $\varepsilon_i$ of a single layer $i$ only. The characterizing parameter $p_i$ may e.g. be equal to the permittivity $\varepsilon_i$ (or to the real or imaginary part of the same) or to an estimate of the water concentration in layer $i$.

### 2.2. Coplanar Waveguide Transmission Lines

#### 2.2.1. Definition

The term "coplanar waveguide" (CPW) as used in this text and the claims is to be interpreted as an arrangement of an elongate center strip electrode between and at a distance from two ground electrodes. The signal electrode is much longer than it is wide. The signal and ground electrodes are mounted to the same surface of a nonconducting support. Optionally, a further ground electrode may be located on the opposite side of the support (an arrangement called "conductor-backed coplanar waveguide", CBCPW). The electrodes may extend along a straight line, or they may be curved (e.g. in the form of a spiral) or polygonal (e.g. in the form of an L or a U).

Advantageously, the ground electrodes are much wider than the signal electrode as this design provides better field localization and is easier to model.

Furthermore, also advantageously, the width of the electrodes are constant along their longitudinal extension, and also the ground geometry does not change along the CPW, as this design is easiest to model. However, it may also be possible to vary these parameters along the CPW, e.g. by periodically changing the width of the signal electrode.

#### 2.2.2. Examples

Devices with coplanar waveguides are especially suited for being used in the context of the present invention.

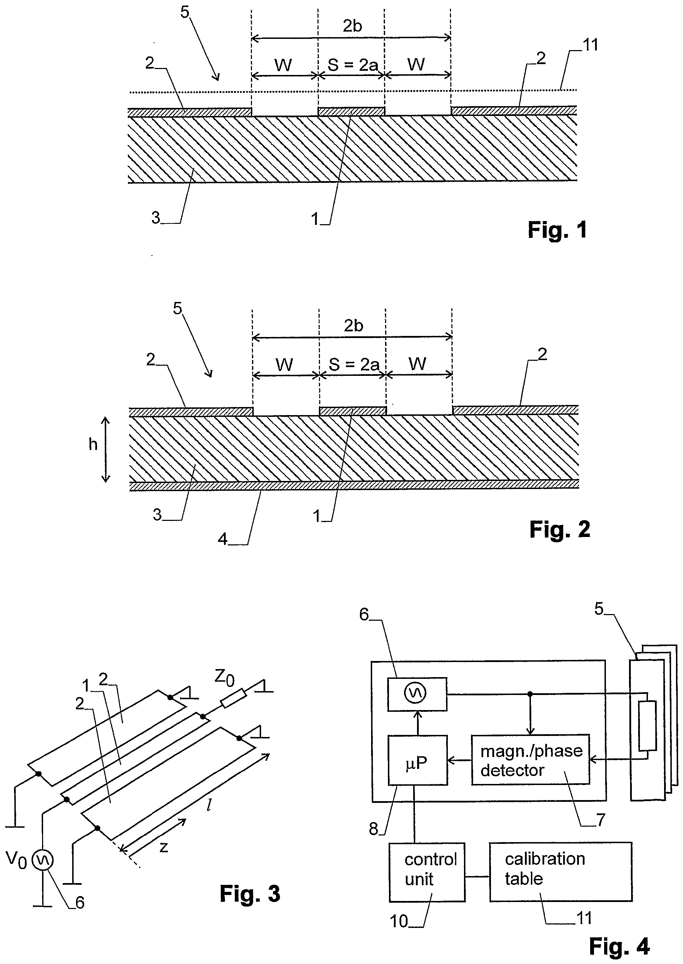

As shown in Fig. 1, an embodiment of a CPW on a dielectric substrate comprises a center strip electrode 1 conductor with (ideally) semi-infinite ground electrodes 2 on either side. Center strip electrode 1 and the ground electrodes 2 are arranged on a dielectric support 3. This structure supports a quasi-TEM mode of propagation. The coplanar waveguide 5 offers several advantages over a conventional microstrip line: First, it simplifies fabrication; second, it facilitates easy shunt as well as series surface mounting of active and passive devices; third, it eliminates the need for wraparound and via holes, and fourth, it reduces radiation loss. Furthermore the characteristic impedance is determined by the ratio of $a/b$, so size reduction is possible without limit, the only penalty being higher losses. In addition, a ground plane exists between any two adjacent lines; hence cross talk effects between adjacent lines are very weak.

The quasi-TEM mode of propagation on a CPW 5 has low dispersion and, hence, offers the potential to construct wide band circuits and components.

Coplanar waveguides can be broadly classified as follows:

- Conventional CPW
- Conductor backed CPW
- Micromachined CPW

In a conventional CPW, the ground planes are of semi-infinite extent on either side. However, in a practical circuit the ground electrodes are made of finite extent. The conductor-backed CPW, as shown in Fig. 2, has an additional bottom ground electrode 4 at the surface of the substrate 3 opposite to electrodes 1 and 2. This bottom ground electrode not only provides mechanical support for the substrate but also acts as a heat sink for circuits with active devices. It also provides electrical shielding for any circuitry below support 3. A conductor backed CPW is advantageously used within this work.

As shown in dotted lines in Fig. 1, the electrodes 1, 2 may optionally be covered by a non-conductive cover layer 11 of known thickness and known dielectric properties. Such a cover layer can be used to avoid any possible electro-chemical effects at the electrodes, and it can also be used to change the effective penetration depths of the fields into the tissue.

### 2.3. Forward Problem for Conductor-Backed CPW (CBCPW)

In the following, the CBCPW 5 of Fig. 2 will be considered. The signal line has the width *S* and the gap width between signal and ground electrodes is *W*. The following annotations are used as well: *S = 2a* and *S + 2W = 2b.*

First, the forward problem of the transmission line has to be solved, i.e. the calculation of the effective permittivity $\varepsilon_{\text{eff}}$ of the system depicted in Fig. 2. Usually, the shown configuration is used with air on top within the high-frequency systems ($\varepsilon_{r1}$ = 1). In measurement applications, the material under test (MUT) with permittivity $\varepsilon_x$ is placed on top of the transmission line ($\varepsilon_{r1} = \varepsilon_x$).

In order to be able to analytically state some simple relationships for the CPWs, a number assumptions and approximations have to be made. The main assumption is that the quasi-TEM (transversal electro-magnetic) wave propagation is dominant on the transmission line. This assumption implies that the losses in the metal strips and dielectric materials are low. This, of course, is not the case for human tissues. However, the analytic expressions allow to quickly analyze the sensor functionality before proceeding to the rigorous computer-aided full-wave analysis.

Based on this approximation, the analysis of Wen [1] can be expanded to the structure under consideration employing the procedure proposed by Gevorgian [2].

The effective permittivity as seen by the transmission line in Fig. 2 can be expressed by

$$\varepsilon_{eff} = 1 + q_1(\varepsilon_r - 1) + q_2(\varepsilon_x - 1); \qquad (2.1)$$

with $\varepsilon_r$ being the permittivity of support 3 and wherein

$$q_1 = \cfrac{1}{1 + \cfrac{K(k_0)}{K(k_0')} \cdot \cfrac{K(k')}{K(k)}}; \qquad (2.2)$$

$$q_2 = \cfrac{1}{1 + \cfrac{K(k_0')}{K(k_0)} \cdot \cfrac{K(k)}{K(k')}} \cdot \qquad (2.3)$$

The functions *K(x)* in Eqs. 2.2 and 2.3 are the complete elliptic integrals of the first kind. Re-arranging the Eqs.2.2 and 2.3, the effective permittivity of the system can be stated as:

$$\varepsilon_{eff} = \varepsilon_r \cdot q_1 + \varepsilon_x \cdot q_2 \qquad (2.4)$$

$$= \cfrac{\varepsilon_r}{1 + \cfrac{K(k_0)}{K(k_0')} \cdot \cfrac{K(k')}{K(k)}} + \cfrac{\varepsilon_x}{1 + \cfrac{K(k_0')}{K(k_0)} \cdot \cfrac{K(k)}{K(k')}}; \qquad (2.5)$$

The parameters $k_i$ depend on the structure geometry and are defined as follows:

$$k_0 = \frac{S}{S + 2W} = a/b, \qquad (2.6)$$

$$k_0' = \sqrt{1 - k_0^2}; \qquad (2.7)$$

and

$$k = \frac{\tanh\left(\frac{\pi \cdot a}{2h}\right)}{\tanh\left(\frac{\pi \cdot b}{2h}\right)}, \tag{2.8}$$

$$k' = \sqrt{1 - k^2}. \tag{2.9}$$

The characteristic impedance of the transmission line can then be calculated to:

$$Z_L = \frac{60\pi}{\sqrt{\varepsilon_{eff}}} \cdot \left[\frac{K(k)}{K(k')} + \frac{K(k_0)}{K(k_0')}\right]^{-1}. \tag{2.10}$$

### 2.4. Permittivity Measurements Using CPW Lines

Due to several boundary conditions, such as size, form (planarity), bandwidth of operations, simplicity, non-invasiveness, the transmission-line technique is employed here. This technique is based on the fact that the wave propagation along the line is strongly affected by the permittivity of the dielectric material supporting the line. There are numerous publications which describe various aspects of the utilisation of this method for material characterisation from theoretical considerations of the inverse problem [4, 5] to practical sensor implementations [6 - 8].

Using Eq. (2.4), the inverse problem of the determination of the permittivity $\varepsilon_{r1} = \varepsilon_x$ can be solved using the following equation:

$$\varepsilon_x = \frac{1}{q_2}\left(\varepsilon_{eff} - \varepsilon_r \cdot q_1\right), \tag{2.11}$$

where $q_1$ and $q_2$ are defined by Eqs. (2.2) and (2.3), respectively.

2.4.1. Theory of the Sensor Operations

The unknown effective permittivity $\varepsilon_{eff}$ of the measurement system has to be determined experimentally. As described in the preface to this subsection, there are various methods to do so. Fig. 3 demonstrates graphically an advantageous method. A generator 6 provides a sinusoidal RF signal, which is applied to the input of center strip electrode 1. The voltage $V(l)$ at the output of the center strip electrode 1 is measured. The propagating wave is attenuated and its velocity is reduced due to the higher permittivity of the medium in comparison to the free space. The following equation describes the voltage variation along the transmission line:

$$V(z) = V_p(z) \cdot e^{-\gamma \cdot z} + V_r(z) \cdot e^{\gamma \cdot z}, \tag{2.12}$$

where $V_p(z)$ and $V_r(z)$ are the amplitudes of the signals propagating forth and back along the line. In case of the line termination with the specific impedance (usually 50Ω), the amplitude $V_r(z)$ of the reflected wave vanishes. Then, the voltage at the termination can be stated as

$$V(l) = V_0 \cdot e^{-\gamma \cdot l}. \tag{2.13}$$

The transfer function of the transmission line is then

$$H = e^{-\gamma \cdot l} \cdot e^{j \cdot \omega \cdot t} = e^{-\gamma \cdot l + j \cdot \omega \cdot t} = \tag{2.14}$$

7

$$= e^{-\alpha \cdot l} \cdot e^{j \cdot (\omega \cdot t - \beta \cdot l)} \qquad\qquad (2.15)$$

Comparing the transfer function with the forward transmission coefficient $S_{21} = |S_{21}| \cdot e^{-j \cdot \varphi}$, the following relationships for the attenuation and the phase of the measured signal at the CPW output can be defined:

$$\alpha = -\frac{|S_{21}|}{l \cdot 20 \log e}, \qquad\qquad (2.16)$$

$$\varphi = 360° \cdot l \cdot f \sqrt{\mu_0 \varepsilon_0} \cdot \sqrt{\varepsilon_{eff}} \ . \qquad\qquad (2.17)$$

It has to be noted at this point that the measured phase delay $\varphi_m$ is usually higher than the value calculated in Eq. (2.17) due to the non-ideal matching of the measurement transmission line.

Combining Eqs. (2.11 and (2.17), the unknown permittivity $\varepsilon_x$ of the material under test can be defined as

$$\varepsilon_x = \frac{1}{q_2} \left( \left[ \frac{\varphi_0 - \varphi_m}{360°} \cdot \frac{1}{l \cdot f \cdot \sqrt{\mu_0 \varepsilon_0}} \right]^2 - \varepsilon_r \cdot q_1 \right), \qquad\qquad (2.18)$$

where $\varphi_m$ is the measured phase delay by the sensor hardware in degrees, which differs from the phase delay over the transmission line. The base phase shift $\varphi_0$ is a constant defined by the sensor hardware. It has to be determined by a calibration procedure as described later.

2.4.2. Sensor Hardware

Fig. 4 shows the basic block diagram of the measurements system. A microwave signal is provided by an AC signal generator 6 and then applied to a first end (input end) of signal line 1 of coupling structure 5, which is brought in contact with the skin of a living human or non-human mammal. Coupling structure 5 is a CPW, in particular a CBCPW as described above, with the signal being applied as shown in Fig. 3. Fig. 4 schematically shows that there can be several such coupling structures.

The voltage at the second end (output end) of center strip electrode 1 of coupling structure 5 is fed to a magnitude/phase detector 7. In the present embodiment, this circuit compares the input and output signals of center strip electrode 1 and generates one or two DC signals, whose voltage is proportional to the magnitude ratio and/or phase difference between them. A microcontroller 8 digitizes and stores the measured data, which then can be used as the basis for calculations of the measure of interest. This sensor system is basically a simplified VNA (Vector Network Analyzer) on a board measuring the magnitude and phase of the forward transmission coefficient $S_{21}$. Detector 7 and microcontroller 8 together form a measuring unit for measuring the "measured parameter" $m_i$ of each CPW.

Further, a control unit 10 is provided for processing the measured parameters $m_i$ and for calculating the characterizing parameters $p_i$ therefrom. Control unit 10 may be implemented as part of microcontroller 8 or it may be a separate unit, such as an external computer.

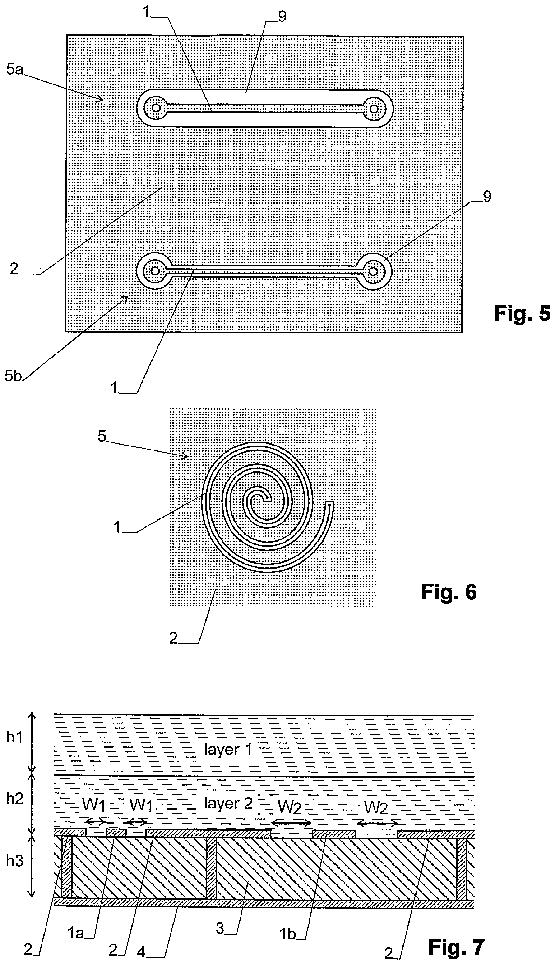

When several CPWs are part of the sensor device, a single signal generator 6 as shown in Fig. 5 can be used for feeding a common signal to all of them such that all CPWs are in operation at the same time. Alternatively, signal generator 6 may be adapted to subsequently feed a signal to each one of the CPWs such that the CPWs are operated in sequence, thereby minimizing crosstalk. Similarly, a measuring unit with several magnitude/phase detectors 7 may be provided, i.e. one detector 7 for each CPW, or a single magnitude/phase detector 7 can be switched between the output ends of the CPWs to sequentially measure the signals from all of them.

### 2.5. Electrode geometries

It has been mentioned that the device is not limited to using straight CPWs. Nor can it use, for obvious reasons, infinitely long CPWs. Fig. 5 shows the design of an advantageous device with two CPWs of different geometry on a single support. In this figure, shaded areas denote the areas covered by center strip electrode 1 and the ground electrodes 2.

The device of Fig. 5 carries two CPWs 5a, 5b that have different gap widths W and therefore generate electrical fields having different penetration within the sample to be measured. CPW 5a has larger gap width W than CPW 5b.

As can be seen, the ground electrodes 2 are formed by a single, structured metal electrode, with each center strip electrode 1 being arranged in an opening 9 of said metal electrode.

As mentioned, the CPW does not necessarily have to extend along a straight line, but may also be curved. An example of a CPW having the form of a spiral is shown in Fig. 6.

In general, though, the cross section of the CPW (as shown in Figs. 1 and 2) should be invariant along the extension z of the center strip electrode, such that the impedance Z does not vary along extension z. Otherwise, more complex models are required for the system modelling.

### 3. Inverse problem for CBCPW

This section describes the detailed procedure derived to calculate the unknown value of the MUT (= Material Under Test) permittivity using a CPW-based device. First, a calibration procedure will be described. This procedure was designed to calculate the unknown parameters of the measurement system or parameters that were intentionally considered to be unknown. Then a mathematic description is defined, which is aimed at calculating the unknown permittivity of the MUT. Finally, a two-layer system is investigated. Using some approximations, both unknown permittivity values are calculated from measured results ("inverse profiling").

Some assumptions have to be made in order to be able to analytically describe the measurements of the permittivities employing the proposed sensor structure.

- Quasi-TEM wave propagation as described in Sec. 2.2
- The capacitance values introduced by the radial signal junctions (i.e. the junctions at the ends of center strip electrode 1) can be accounted for by an additional length of the transmission lines. I.e., an ideal CPW with $l_{eff} > l$ describes the behavior of the transmission line. This is a very valid assumption as the phase delay can be later easily be accounted for by the open coaxial-capacitance models.
- In the case of two-layer MUT, the EM field induced by the transmission line with the shorter $W = \Delta GS$ distance is mostly confined within the first layer, i.e. permittivity variation within the second (deeper) layer does not affect the propagation properties of the transmission line. This condition can be assumed during the first stage of the mathematical considerations. The penetration depth is a very critical value as it depends on the material parameters, sensor geometry, *and* frequency of operations.

It must be noted that the above assumptions simplify an analytical analysis of the system. The invention, though, does not necessarily rely on them. If the assumptions are not met, the system can e.g. still be modeled numerically if no analytical description can be derived.

### 3.1. Calibration Procedure

In the following, an example of a calibration procedure for a geometry as shown in Fig. 2 (CBCPW) is described. The procedure was then tested on a device having copper electrodes, copper vias (lead throughs) and a Rogers RO4350b support ($\varepsilon_r$ = 3.66). The device had two CPWs having different widths W.

Eq. (2.18) is repeated below as (3.1). This relationship defines the unknown permittivity from the phase delay $\varphi_m$ measured by the sensor system.

$$\varepsilon_x = \frac{1}{q_2}\left(\left[\frac{\varphi_0 - \varphi_m}{360°} \cdot \frac{1}{l_{eff} \cdot f \cdot \sqrt{\mu_0 \varepsilon_0}}\right]^2 - \varepsilon_r \cdot q_1\right) \tag{3.1}$$

In the above equation, $q_1$ and $q_2$ denote the so-called "filling factors" of the substrate and the unknown material, respectively. The value $\varphi_0$ is a constant base phase shift (for constant frequency and line dimensions) defined by the system, *f* is the frequency of operation, $\mu_0$ and $\varepsilon_0$ are physical constants for absolute permeability and permittivity of the free space, respectively. Finally, $l_{eff}$ is the effective length of the measurement transmission line. This length equals to the geometrical length *l* in the case of ideal CPW. In the current case of a real sensor system, the measured phase delay is slightly higher than it would be theoretically expected. This effect is assumed to be accounted for by an effective length $l_{eff} > l$ as discussed above.

For fixed dimension and frequency, Eq. (3.1) can be rewritten in the following form

$$\varepsilon_x = C_1 + C_2 \cdot \left(\varphi_0 - \varphi_m\right)^2 \tag{3.2}$$

The three unknown constants $C_1$, $C_2$, and $\varphi_0$ only depend on the sensor geometry and the operating frequency. They can be easily found if at least three measurements on materials with known permittivities (instead of the MUT) are performed. Assuming that the known calibration materials have permittivity values of $\varepsilon_1$, $\varepsilon_2$ and $\varepsilon_3$, and the corresponding measured phase values are $\varphi_1$, $\varphi_2$ and $\varphi_3$ respectively, the calibration constants can be defined as follows:

$$\varphi_0 = \frac{1}{2} \cdot \frac{\left(\varepsilon_3 - \varepsilon_2\right)\cdot \varphi_1^2 - \left(\varepsilon_3 - \varepsilon_1\right)\cdot \varphi_2^2 + \left(\varepsilon_2 - \varepsilon_1\right)\cdot \varphi_3^2}{\left(\varepsilon_3 - \varepsilon_2\right)\cdot \varphi_1 - \left(\varepsilon_3 - \varepsilon_1\right)\cdot \varphi_2 + \left(\varepsilon_2 - \varepsilon_1\right)\cdot \varphi_3} \tag{3.3}$$

$$C_2 = \frac{\varepsilon_2 - \varepsilon_1}{\left(\varphi_0 - \varphi_2\right)^2 - \left(\varphi_0 - \varphi_1\right)^2} \tag{3.4}$$

$$C_1 = \varepsilon_1 + \frac{\varepsilon_2 - \varepsilon_1}{1 - \left(\dfrac{\varphi_0 - \varphi_2}{\varphi_0 - \varphi_1}\right)^2} \tag{3.5}$$

The derived calibration procedure has to be performed only once for each single sensor. It has only to be repeated if the hardware (either electronics or the coupling structure) is changed. Using the found calibration constants, the permittivity of an unknown material can be calculated easily.

*Example*

A sensor having two CPW transmission lines width gap widths 0.1 mm and 0.2 mm, respectively, and the length of 25 mm was calibrated at the frequency of 0.8 GHz using air ($\varepsilon = 1$), ethanol ($\varepsilon = 16.34$) and distilled water ($\varepsilon = 79.00$). Using eqs. (3.3) - (3.5) above, the following results were obtained for the parameters $\varphi_0$, $C_1$, $C_2$:

- CPW with W = 0.1 mm: $\varphi_0 = 158.3$, $C_1 = -4.104$, $C_2 = 0.00355$
- CPW with W = 0.2 mm: $\varphi_0 = 156.3$, $C_1 = -5.859$, $C_2 = 0.00266$

### 3.2. "Inverse Profiling"for Two-Layer Problem

Fig. 7 demonstrates a configuration for determining the permittivity values of two layers 1 and 2. In order to tackle this problem, at least two measurements have to be performed. The ansatz in this work is to use at least two CPWs with different values of the ground-to-signal distance W. In the embodiment of Fig. 7, the first CPW has a center strip electrode 1a and the second one a center strip electrode 1b, with corresponding gap distances *W1* and *W2*, respectively.

Furthermore, for simplicity, an additional condition should advantageously be fulfilled, which was already defined at the beginning of the section: the field induced by the transmission line with the shorter W = $\Delta GS$ distance (i.e. 'short') is confined within the layer 2, i.e. permittivity variation within the deeper layer 1 does not affect the propagation properties of the transmission line with smaller *W.* In the following subsections, a procedure is described that allows to calculate the desired unknowns.

3.2.1. Forward Problem of CBCPW with a Two-Layer MUT

First, the forward problem, i.e. the calculation of the effective relative permittivity $\varepsilon_{eff}$ of the described structure, is solved. This is performed employing the conformal-mapping technique defined by Veyres and Hanna [9] for finite CPW and modified by Bedair and Wolff [4] for multi-layer structures. The described considerations are only valid if the permittivity of the supporting material is lower than the unknown permittivities (which is the case for biological tissues).

The effective relative permittivity of the structure depicted in Fig. 7 can, analogously to Eq. (2.1), be stated as:

$$\varepsilon_{eff} = \varepsilon_1 \cdot q_1 + \varepsilon_2 \cdot q_2 + \varepsilon_3 \cdot q_3 \tag{3.7}$$

Again, $q_1$, $q_2$, $q_3$ are the filling factors for the layers 1 - 3, respectively. The approach uses an exact expression for the characteristic impedance

$$Z_0^a = \frac{1}{c_0 \cdot C_t^a} \qquad (3.8)$$

where $c_0$ = 2.9979·10^8 m/s is the speed of light and $C_t^a$ is capacitance per unit area if the air-filled capacitors are considered ($\varepsilon_1 = \varepsilon_2 = \varepsilon_3 = 1$). Then, the characteristic impedance of the considered transmission line can be stated as:

$$Z_0 = \frac{Z_0^a}{\sqrt{\varepsilon_{eff}}} \qquad (3.9)$$

The air-filled capacitors can be defined as:

$$C_i^a = 2\varepsilon_0 \frac{K(k_i)}{K(k_i')} \quad \text{with} \quad (i = I, II, III) \qquad (3.10)$$

with $K(k_i)$ and $K(k_i')$ as the complete elliptic integral if the first kind similar to the Eq. (2.2) and (2.7) and (2.9). In our particular case, the $k_i$ can be defined as follows:

$$k_I = \frac{S}{S + 2W} = a/b \; ; \qquad (3.11)$$

$$k_{II} = \frac{\sinh\left(\dfrac{\pi \cdot a}{2h_2}\right)}{\sinh\left(\dfrac{\pi \cdot b}{2h_2}\right)} \; ; \qquad (3.12)$$

$$k_{III} = \frac{\tanh\left(\dfrac{\pi \cdot a}{2h_3}\right)}{\tanh\left(\dfrac{\pi \cdot b}{2h_3}\right)} \qquad (3.13)$$

The following values can be determined from the geometry and assumptions made by Veyres and Hanna [9]:

$$C_t^a = C_I^a + C_{III}^a \qquad (3.14)$$

$$q_3 = \frac{C_{III}^a}{C_t^a} \qquad (3.15)$$

$$q_2 = \frac{C_{II}^a}{C_t^a} \qquad\qquad (3.16)$$

$$q_1 = \frac{C_I^a - C_{II}^a}{C_t^a} \qquad\qquad (3.17)$$

Using the above expressions, the forward problem depicted in Fig. 7 reduces to

$$\varepsilon_{eff} = \frac{C_I^a - C_{II}^a}{C_I^a + C_{III}^a} \cdot \varepsilon_1 + \frac{C_{II}^a}{C_I^a + C_{III}^a} \cdot \varepsilon_2 + \frac{C_{III}^a}{C_I^a + C_{III}^a} \cdot \varepsilon_3 \qquad\qquad (3.18)$$

with $C_i^a$ defined by Eq. (3.10).

3.2.2. A Method for the Solution of the Inverse Problem

In the following a possible solution for the inverse problem is presented. It is based on several assumptions, which will be defined within the course of explanation. The coupling structure used consists of two conductor-backed coplanar waveguides as shown in Fig. 7. The described solution comprises the following steps.

*Calibration of both sensor configurations*

This has to be performed according to the procedure described in Sec. 3.1. The calibration materials can be, for example: Air($\varepsilon_1 = 1$), ethanol ($\varepsilon_2$), and distilled water ($\varepsilon_3$). Using Eqs. (3.3) - (3.5), the following two sets of calibrations constants for each frequency value can be defined:

$\varphi_{0s}$, $C_{2s}$, $C_{1s}$, for the 'short' CPW (*W* small) and
$\varphi_{0l}$, $C_{2l}$, $C_{1l}$, for the 'long' CPW (*W* large)

*Permittivity of layer 2*

Under the above assumption that the field induced by the 'short' CPW (= CPW with smaller gap width *W*) is confined within the layer 2, the permittivity of this layer can be calculated to be

$$\varepsilon_2 = C_{1s} + C_{2s} \cdot \left(\varphi_{0s} - \varphi_{ms}\right)^2 \qquad\qquad (3.19)$$

with $\varphi_{ms}$ being the phase-delay value measured over the 'short' CPW applied to the unknown material (MUT).

*Effective permittivity as "seen" by the 'long' CPW*

The following step is the calculation of the effective permittivity as "seen" by the 'long' CPW (= CPW with larger gap width *W*). It is the dielectric characteristic of the hypothetical material mixture between layers 1 and 2 that defines the propagation properties of the transmission line with the wide ground-to-signal distance. The relative permittivity of this material mixture can be calculated by Eq. (3.20)

$$\varepsilon_l = C_{1l} + C_{2l} \cdot \left(\varphi_{0l} - \varphi_{ml}\right)^2, \qquad\qquad (3.20)$$

where $\varphi_{ml}$ is the phase-delay value ascertained by the sensor over the 'long' CPW applied to (MUT).
In order to be able to define the effective permittivity of the assumed material mixture, let's assume that the layers 1 and 2 are merged and describe a material layer with infinite thickness and relative permittivity $\varepsilon_l$. For this new two-layer system with the single-layer MUT, the effective permittivity can be written as:

$$\varepsilon_{eff,l} = q_{2l2} \cdot \varepsilon_l + q_{3l2} \cdot \varepsilon_3 \qquad (3.21)$$

$\varepsilon_l$ is defined in (3.20), $\varepsilon_3$ is the relative permittivity of the supporting substrate, and the filling parameters $q_{2l2}$ and $q_{3l2}$ can be calculated by Eqs. (3.16) and (3.15), respectively. The corresponding parameters for the determination of the elliptic integrals can be determined:

$$k_{2l2} = a_l \big/ b_l ; \qquad (3.22)$$

$$k_{3l2} = \frac{\tanh\left(\dfrac{\pi \cdot a_l}{2h_3}\right)}{\tanh\left(\dfrac{\pi \cdot b_l}{2h_3}\right)}; \qquad (3.23)$$

$$k_i' = \sqrt{1 - k_i^2}, \qquad i = 2l2,\, 3l2 \qquad (3.24)$$

$a_l$ and $b_l$ are the geometric parameters of the 'long' CPW.

*Inverse Profiling of a Two-Layer MUT*

Now, let's consider the original measurement problem depicted in Fig. 7. The permittivity value $\varepsilon_1$ can be calculated from Eq. (3.18):

$$\varepsilon_1 = \frac{1}{q_{1l}}\left(\varepsilon_{eff,l} - q_{2l} \cdot \varepsilon_2 - q_{3l} \cdot \varepsilon_3\right) \qquad (3.25)$$

Considering the fact that $q_{3l} = q_{3l2}$ and using Eq. (3.21), the expression (3.25) reduces to:

$$\varepsilon_1 = \frac{1}{q_{1l}}\left(q_{2l2} \cdot \varepsilon_l - q_{2l} \cdot \varepsilon_2\right) \qquad (3.26)$$

According to Eqs. (3.10)-(3.13) and (3.16), $q_{2l}$ is defined as

$$q_{2l} = \frac{\dfrac{K(k_{2l})}{K(k_{2l}')}}{\dfrac{K(k_{1l})}{K(k_{1l}')} + \dfrac{K(k_{3l})}{K(k_{3l}')}} \qquad (3.27)$$

with parameters $k_i$ and $k_i'$ obtained as follows:

$$k_{1l} = a_l \big/ b_l ; \qquad (3.28)$$

$$k_{2l} = \frac{\sinh\left(\dfrac{\pi \cdot a_l}{2h_2}\right)}{\sinh\left(\dfrac{\pi \cdot b_l}{2h_2}\right)}; \tag{3.29}$$

$$k_{3l} = k_{3l2} = \frac{\tanh\left(\dfrac{\pi \cdot a_l}{2h_3}\right)}{\tanh\left(\dfrac{\pi \cdot b_l}{2h_3}\right)}; \tag{3.30}$$

$$k_i' = \sqrt{1 - k_i^2} \tag{3.31}$$

At this point, it has to be mentioned that the value of $h_2$ is not known. It is only assumed here that this parameter describes the "penetration" depth of the EM-field induced by the 'short' transmission line. Generally, this value depends on the dimension of the transmission line, parameters of the unknown material, and frequency of operation.

3.2.3. Summary

The derived procedure allows to calculate the two unknown permittivity values for a two-layer material under tests employing the CPW sensor with two transmission lines with different ground-to-signal distance dimensions. The procedure comprises the following steps:

   (a) Calibrate the device by carrying out test measurements with single layer systems of known substances, such as air, ethanol and distilled water. This provides the calibration constants $\varphi_{0s}$, $C_{2s}$, $C_{1s}$, for the CPW with smaller gap width $W$ and $\varphi_{0l}$, $C_{2l}$, $C_{1l}$, for the CPW with larger gap width $W$. This calibration has to be performed just once for every hardware configuration.
   (b) Apply the device to the surface of an unknown two-layer system. Calculate the dielectric constant $\varepsilon_2$ of layer 2 using Eq. (3.19) and the dielectric constant $\varepsilon_1$ of layer 1 using Eqs. (3.26) and (3.20).

## 4. Applications

### 4.1. Test measurements

Various test measurements were carried out with different test persons. The used procedure comprised a series of measurements with a test device. The test device was operated at a frequency of 0.8 GHz and had two conductor-backed CPW waveguides as shown in Fig. 7. It had a first CPW with a gap width $W$ = 0.1 mm and a second CPW with a gap width $W$ = 0.2 mm.
The test procedure comprised a first reference measurement with the device exposed to air (not to skin) at the start and a first measurement with the device applied to the skin region to be tested after two minutes, the application of the agent to the skin region after three minutes and during three minutes, and subsequent measurements at various times by again applying the device to the skin region. Before the first measurements after application of the agent, the remaining agent was gently wiped away from the skin using a soft tissue. Each measurement had a duration of five seconds, whereupon the device was removed from the skin. Every time, the measurements were repeated five times with a 10 second break between measurements to avoid the occlusion effect because of changes caused by the decrease in transepidermal water loss that occurs when the skin is covered.
Using the techniques described in section 3.2.2, the permittivities $\varepsilon_1$ and $\varepsilon_2$ of two layers were calculated from the measured parameters. It was assumed that the penetration depth of the fields in the skin region is roughly equal to the gap widths $W$ of the two CPWs used, thus it was assumed that the topmost layer (layer 2) has a thickness of 0.1 mm (therefore corresponding to the stratum corneum, while the inner layer (layer 1) basically corresponds to the epidermis. In other words, it was assumed that the skin could be modeled by a two-layer system having a top layer of a thickness of 0.1 mm.

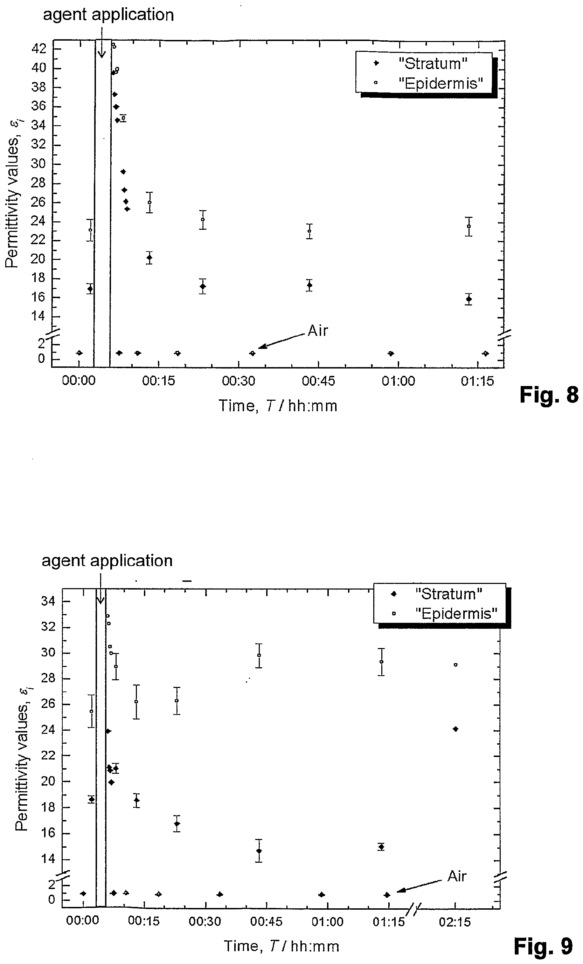

Figs. 8 and 9 demonstrate the time dependence of the permittivity values $\varepsilon_i$ during the test for two test substances. The test substance used in Fig. 8 was the NutraPlus hydration lotion by Galderma, Switzerland. The test substance used in Fig. 9 was the Basodexan creme by Hermal, Germany.

Initially, the application of both crèmes has the same effect on the stratum permittivity: the value rises indicating the increased moisture content only to decrease exponentially. For the NutraPlus lotion, the stratum moisturization returns to the initial value after some 20 minutes. It even falls slightly below it after more than an hour indicating the fact that the skin is dryer than it was previously, thus calling for repetition of the lotion application. In contrast to this behaviour, the permittivity of the skin covered by the Basodexan creme rises again after having reached a minimum after some 45 minutes. This effect seems logical considering that the active agent of Basodexan is urea, which helps to enforce the water-proof barrier in the stratum corneum. The moisture from the dermis is then prevented from evaporating at the surface.

An interesting behaviour can be observed for the epidermis permittivity. It also initially rises very quickly after the creme application only to return to the same level after some 15 min. As the moisture is very unlikely to diffuse to the epidermis so quickly, it can be assumed that the initial rise is attributed to the thin film of the creme at the skin surface, which evaporates as the time progresses. The inverse-profiling procedure used in this example with two CPW lines describes two-layer materials only and doesn't account for three layers (creme film, stratum, epidermis).

After 15 minutes, the permittivity of the skin with the NutraPlus lotion remains the same level until the end of the measurement. In contrast to that, the Basodexan creme causes an increase of the epidermis permittivity after some 40 minutes while the stratum permittivity still decreases. The increased relative permittivity of the epidermis and, thus, its moisturization level remain at the same increased value until the end of the measurement for more than 2 hours after the creme application.

As this analysis shows, the measured permittivities allow the interpretation of the mechanisms of moisture transport in the skin in considerable detail, therefore allowing to characterize the effect of the skin treatment agents on the skin.

### 4.2. Characterizing parameters

The characterizing parameters $p_i$ used in the test measurements of above section 4.1 were the permittivities $\varepsilon_1$, $\varepsilon_2$ of the layers of a two-layer model. Alternatively, an estimate of the water contents of the skin layers can be calculated. Since water makes a major contribution to the permittivity value of the tissue, the knowledge of the permittivity values of the different layers of the tissue allows one to provide an estimate of water content for the given layers. In a simple model, based on Kraszewski mixture formula [10], it can be assumed that the volume fraction $p_i$ of water in a material, tissue, or emulsion can be expressed as a function of measured permittivity $\varepsilon_i$ and permittivities (real part of) of water ($\varepsilon_1$) and dry matter ($\varepsilon_2$):

$$\mathrm{p_i} = \frac{\varepsilon_i^{0.5} - \varepsilon_2^{0.5}}{\varepsilon_1^{0.5} - \varepsilon_2^{0.5}} \tag{4.1}$$

Additional capability of the described sensor and procedure is the determination of the water content (or content of other matter with known permittivity) in different layers not necessary laying on the surface. I.e. using the described system, it is possible to make depth profiling of the material under investigation assumed to be composed of two materials or two material groups.

In general, each characterizing parameter depends on the permittivity $\varepsilon_i$ of a single skin layer *i* of the investigated skin region, i.e. the techniques described in section 3.2 are used for separating the contributions of the various layers to the effective permittivities as seen by the electric fields.

Advantageously, the characterizing parameters $p_i$ are determined repetitively, at different times after application of the agent, which provides more detailed information about the moisture transport mechanisms as shown in section 4.1, in particular by comparing the characteristic parameters $p_i$ recorded at different times and/or for different layers *i*.

### 4.3. Frequency

An important parameter of the measurements described here is the frequency of the applied fields. In general, CPW-type sensors operated in transmission, as described here, are especially suited for measurements in the range of approximately 50 MHz to 100 GHz. For too low frequencies, the necessary line length would become too long.

The device can also carry out measurements at more than one frequency, either concurrently or consecutively.

### 4.4. Electrode set dimensions

The primary factor determining the reach of the field of an electrode set into the body tissue is its gap width *W*. Electrode sets having a sufficiently large range of electrode distances should be incorporated into the device for obtaining spatially resolved measurements of each skin layer having dielectric properties of interest.

In particular, at least one electrode set should have an electrode distance of 100 μm or less in order to obtain a measurement specific for the stratum corneum.

Similarly, at least one other electrode set should have a gap width *W* of at least 0.1 mm in order to obtain a measurement indicative of epidermis or dermis properties. In particular, the gap width of this electrode set should be in a range of 0.1 mm to 0.2 mm since an electrode set with a larger gap width will tend to create a field reaching into the dermis.

If a characterizing parameter for the dermis is required, a set of electrodes having a mutual distance of at least 1 mm should be provided.

### 4.5. Combination with other characterization methods

The characterizing parameters $p_i$ as obtained above can be combined with further characterization methods to improve the understanding of the effects of the skin treatment agent. Examples of such methods are described in the following.

- *Measuring an optical reflection or transmission of the skin region*: A light wave is directed onto the skin region and the amount of reflected light or the amount of transmitted light is used for assessing the state of the skin. Suitable methods are e.g. described in US 6 070 092. The result of these measurements can e.g. be used for determining if the skin is in a state suitable for testing an agent. Also, optical transmission and reflection measurements, in particular in the near infrared, allow to detect an amount of water in the skin as described in US 6 442 408.
- *Measuring a temperature of the skin region:* The device can e.g. be equipped with a temperature sensor for measuring the temperature of the skin region. This temperature is an important parameter since the permittivity of water changes with temperature. The temperature can therefore be used to calculate the moisture content of a given layer *i* from its permittivity $\varepsilon_i$ and the measured temperature, e.g. by varying the parameters $p_{i0}$ and $p_{i1}$ of Eq. (4.1) as a function of temperature. The temperature sensor can be a conventional contacting temperature sensor, or it can be based on infrared emission measurements, or it can use a dielectric measurement as described in US 2004/0240512.
- *Measuring a conductivity of said skin region for a frequency below 1 MHz:* It has been found that the conductivity of a skin region at such low frequencies are primarily determined by surface moisture, in particular sweat. By measuring this conductivity, the state of the skin can therefore be assessed in more detail. For example, it can be ruled that a skin region having a low frequency conductivity exceeding a given threshold value is unsuitable for characterizing an agent.
- *Measuring an environmental air humidity:* The humidity of environmental air is an important parameter affecting the moisture transport in the topmost skin layers. Hence, by measuring environmental air humidity, it becomes possible to ensure that characterization measurements are carried out under comparable conditions, e.g. by only carrying out the test if the humidity lies within a given range.
- *Measuring an evaporation from the skin region:* Various methods are known for measuring skin evaporations, e.g. as described in US 6 966 877. An knowledge of the skin evaporation rate can provide further insights into the moisture transport mechanisms of the topmost skin layer. While the above methodology allows to assess the moisture levels in the skin layers at a given time, it provides only indirect insight into the rate of moisture flow. By measuring skin evaporation rates, a direct measure of the transport rate off the top layer into the surrounding atmosphere is obtained, which in turn allows to assess the flow rates between two individual skin layers.

### 4.6. General remarks

It must be noted that the present invention provides for a number of steps to calculate the "characterizing parameters" as defined above. The characterizing parameters are quantitative values that can be calculated. They then have to be used for the characterization of the agent. This characterization requires additional steps, which are of a more qualitative, interpretive nature and depend on the type of characterization that is required. An example of such further, interpretive steps is given in section 4.1, but these steps will vary depending on the questions to be answered by the characterization. For example, a deodorant may be investigated as to how it suppresses sweat generation, while the characterization of a moisturizing crème will focus on how the moisture in the skin layers increases or the drying of the skin due to the use of soaps or cleansing agents.

A primary application is a characterization of the agents by means of an observation of the evolution of water concentrations (as determined from the characterizing parameters) as a function of time of one or more skin layers after application of the agent.

Depending on what characterization is required, the skilled person will e.g. be interested in the moisture development in a single layer or in several layers.

The skilled person will be readily able to determine the qualitative, interpretive steps depending on the questions that he needs answers for.

### 4.7. General remarks

The number $N$ of CPWs having different gap widths $W$ depends on the number of tissue layers that are to be observed. For any depth-resolved measurement, $N$ must be larger than 1. In the above examples, two CPWs were used, but the number $N$ can easily be increased to higher values, such as 4 or more. In that case, the method for inverse profiling can be generalized to make a depth profile of a material having N layers. These layers can also be virtual and have only a theoretical depth. To perform a profiling for more than two layers, one can proceed as follows (1 is the most inner and N is the most top layer):

1. Consider the entire material consisting of two layers (consisting of several layers again). For example, to start profiling of a four-layer system using a measurement system with four CPWs, solve the two-layer problem for layers 4 and 3 using the measurements on the corresponding CPWs (e.g. 4 and 3).
2. In the next step, the parameters of the layer 2 can be calculated employing the measurements on the CPWs 3 and 2. In this case, the layers 4 and 3 are considered to be a single virtual layer 3*.
3. Proceed until all wanted parameters are calculated.

The present invention is set out in the appended claims. The embodiments, aspects or examples according to the present description that do not fall within the scope of said claims are provided for illustrative purposes only and do not form part of the present invention.

*References*

[1] C.P. Wen, "Coplanar Waveguide: A Surface Strip Transmission Line Suitable for Nonreciprocal Gyromagnetic Device Applications," in IEEE Trans Microwave Theory Techn., Dec 1969, pp. 1087-1090.
[2] S. Gevorgian, L.J.P. Linnér, E.L. Kollberg, "CAD Models for Shielded Multilayered CPW," in IEEE Trans Microwave Theory Techn., April 1995, pp. 772-779.
[3] J. Baker-Jarvis, M.D. Janezic, B.F. Riddle, R.T. Johnk, P. Kabos, Ch.L. Holloway, R.G. Geyer, and Ch.A. Grosvenor, Measuring the Permittivity and Permeability of Lossy Materials: Solids, Liquids, Building Materials, and Negative-Index Materials. NIST Technical Note 1536, Boulder, CO: NIST, 2005.
[4] S.S. Bedair and I. Wolff, "Fast, Accurate and Simple Analytic Formulas for Calculating the Parameters of Supported Coplanar Waveguides for (M)MIC's," in IEEE Trans. Microwave Theory Techn, vol. 40, Jan. 1992, pp. 41-48.
[5] M.D. Janezic, D.F. Williams, "Permittivivty Characterization from Transmission-Line Measurements," in IEEE MTT-S Int Microwave Symposium Dig., June 197, pp. 1343-1346.
[6] S.S. Stuchly and C.E. Bassey, "Microwave coplanar sensors for dielectric measurements," in Meas Sci Technol., 1998, pp. 1324-1329.
[7] A. Raj, W.S. Holmes, and S.R. Judah, "Wide Bandwidth Measurement of Complex Permittivity of Liquids using Coplanar Lines," in IEE Trans. Instr. Meas., vol. 50, Aug. 2001.
[8] B. Kang, J. Cho, Ch. Cheon, Y. Kwon, "Nondestructive Measurements of Complex Permittivity and Permeability Using Multilayered Coplanar Waveguide Structures," in IEEE Microwave Wireless Comp. Lett., vol. 15, May 2005
[9] C. Veyres and V.F. Hanna, "Extension of the application of conformal mapping techniques to coplanar lines with finite dimensions," in Int. J. Electron., vol. 48, pp. 47-56, 1980.
[10] A. Kraszewski, S. Kulinski, and M. Matuszewski, "Dielectric properties and a model of biphase water suspension at 9.4 GHz," Journal of Applied Physics 47, no. 4 (April, 1976): 1275-1277.

*Reference figures*

1, 1a, 1b: center strip electrode
2: ground electrodes

3: support

4: bottom ground electrode

5, 5a, 5b: coplanar waveguide

6: signal generator

7: magnitude/phase detector

8: microcontroller

9: opening

10: control unit

11: cover layer

$a$: half width of signal line

$b$: half width of ground electrode distance

$a_l$, $b_l$: geometric parameters of "long" CPW

$c_o$: speed of light

$C_1$, $C_2$: device geometry constants, see Eq. 3.2

$C_I$, $C_{II}$, $C_{III}$: air-filled capacitances, see Eq. 3.10

$C_i^a$: capacitance per unit for air-filled capacitors, see Eq. 3.10

$f$: frequency

$h$, $h3$: height of support

$h1$, $h2$: height of layers of two-layer system (Fig. 7)

$H$: transfer function (eq. 2.14)

$K(x)$: complete elliptic integral function

$k_0$, $k'_0$, $k$, $k$, $k_I$, $k_{II}$, $k_{III}'$: structural parameters, Eqs. 2.6ff, 3.11ff

$l$: length

$l_{eff}$: effective length, taking into account the effect of the signal junctions

$m_i$: measured parameter for layer $i$

$N$: number of layers

$P$, $p_i$: characterizing parameters

$q_1$, $q_2$, $q_3$: filling factors, see Eq. 2.2, 2.3 and 3.7

$S$: width of signal line

$S_{12}$: forward transmission coefficient

$W$, $W_1$, $W_2$, $W_i$: width of gaps between signal line and ground, distance of electrode sets

$V(z)$, $V_p(z)$, $V_r(z)$: voltages along the signal line (eq. 2.12)

$z$: position along center strip electrode

$Z_0$: characteristic impedance

$Z_L$: line impedance

$\Delta GS$: = W, see above

$\varepsilon_0$: absolute permeability

$\varepsilon_1$, $\varepsilon_2$ and $\varepsilon_3$: permittivities of calibration media

$\varepsilon_{eff}$: effective permittivity

$\varepsilon_r$: permittivity of support

$\varepsilon_{r1}$: permittivity of space above CPW

$\varepsilon_x$: unknown permittivity

$\varphi$: phase shift

$\varphi_m$: measured phase shift

$\varphi_0$: base phase shift

$\varphi_1$, $\varphi_2$ and $\varphi_3$: phase shift values measured for calibration media

$\gamma$: damping factor

$\mu_0$: absolute permeability

## Claims

1. A method for characterizing an effect of a non-therapeutic skin treatment agent on skin comprising the steps of

    (a) applying the skin treatment agent to a skin region,
    (b) applying a measuring device to said skin region, said measuring device having several sets of electrodes (1, 2; 1a, 1b, 2), wherein each set comprises at least two electrodes and wherein the electrodes of each set of electrodes have a mutual distance $W_i$ from each other and wherein there are $N > 1$ sets having different mutual distances $W_i$,

(c) generating, by means of said different sets of said electrodes, at least $N$ electrical fields within said skin region, said electrical fields having differing penetration depths into said skin region,

(d) measuring at least $N$ measured parameters $m_i$, wherein each measured parameter $m_i$ depends on an effective permittivity as seen by a different one of said $N$ electrical fields

(e) calculating, from said measured parameters $m_i$, at least one characterizing parameter descriptive of a permittivity of said skin region for a given depth,

wherein said measuring device comprises

- at least one set of electrodes having a mutual distance 0.1 mm or less and
- at least one set of electrodes having a mutual distance of at least 0.1 mm.

2. The method of claim 1 wherein step (e) further comprises calculating, from said measured parameters $m_i$, several characterizing parameters $p_i$, in particular $N$ characterizing parameters $p_i$, for differing depths of said skin region.

3. The method of claim 2 wherein each characterizing parameter $p_i$, depends on a permittivity $\varepsilon_i$ of a single layer $i$ of said skin region.

4. The method of any of the claims 2 or 3 wherein each characterizing parameter $p_i$ is the permittivity $\varepsilon_i$ of a single layer $i$ of said skin region.

5. The method of any of the claims 2 or 3 wherein each characterizing parameter $p_i$ is the water content of a single layer $i$ of said skin region.

6. The method of any of the preceding claims wherein said measuring device comprises at least one set of electrodes having a mutual distance between 0.1 mm and 0.2 mm.

7. The method of any of the preceding claims wherein said measuring device comprises at least one set of electrodes having a mutual distance of at least 1 mm.

8. The method of any of the preceding claims wherein the applied electrical fields have a frequency between 50 MHz to 100 GHz.

9. The method of any of the preceding claims comprising the steps of repetitively determining, at different times after application of the non-therapeutic skin treatment agent, the at least one characterizing parameter.

10. The method of any of the preceding claims comprising the step of measuring an optical reflection or transmission of the skin region.

11. The method of any of the preceding claims comprising the step of measuring a temperature of the skin region, and in particular where a moisture content of a layer $i$ of said skin region is calculated from a permittivity $\varepsilon_i$ of said layer $i$ and said temperature.

12. The method of any of the preceding claims comprising the step of measuring a conductivity of said skin region for a frequency below 1 MHz.

13. The method of any of the preceding claims comprising the step of measuring environmental air humidity.

14. The method of any of the preceding claims comprising the step of measuring an evaporation from the skin region.

15. The method of any of the preceding claims wherein said sets of electrodes are formed by coplanar waveguides.

16. The method of any of the preceding claims comprising the step of determining, from said characterizing parameters, an evolution of water concentrations as a function of time in at least one skin layer after application of the non-therapeutic agent.

**Patentansprüche**

1. Ein Verfahren zum Charakterisieren einer Wirkung eines nicht-therapeutischen Hautbehandlungsmittels auf die Haut, umfassend die Schritte

   (a) Auftragen des Hautbehandlungsmittels auf einen Hautbereich,
   (b) Aufbringen einer Messvorrichtung auf den Hautbereich, wobei die Messvorrichtung mehrere Sätze von Elektroden (1, 2; 1a, 1b, 2) aufweist, wobei jeder Satz mindestens zwei Elektroden umfasst und wobei die Elektroden jedes Satzes von Elektroden einen gegenseitigen Abstand Wi voneinander aufweisen und wobei es N > 1 Sätze mit unterschiedlichen gegenseitigen Abständen Wi gibt,
   (c) Erzeugen von mindestens N elektrischen Feldern innerhalb des Hautbereich mittels der mehreren Sätze von Elektroden, wobei die elektrischen Felder unterschiedliche Eindringtiefen in den Hautbereich aufweisen,
   (d) Messen von mindestens N gemessenen Parametern mi, wobei jeder gemessene Parameter mi von einer effektiven Permeabilität abhängt, die in einem anderen der N elektrischen Felder gesehen wird,
   (e) Berechnen von mindestens einem charakterisierenden Parameter, der eine Permeabilität des Hautbereichs für eine gegebene Tiefe beschreibt, aus den gemessenen Parametern mi,

   wobei die Messvorrichtung umfasst

   - mindestens einen Satz von Elektroden mit einem gegenseitigen Abstand von 0,1 mm oder weniger und
   - mindestens einen Satz von Elektroden mit einem gegenseitigen Abstand von mindestens 0,1 mm.

2. Das Verfahren nach Anspruch 1, wobei Schritt (e) weiter umfasst
   Berechnen, aus den gemessenen Parametern mi, mehrerer charakterisierender Parameter pi, insbesondere N charakterisierender Parameter pi, für unterschiedliche Tiefen des Hautbereichs.

3. Das Verfahren nach Anspruch 2, wobei jeder charakterisierende Parameter pi von einer Permeabilität $\varepsilon_i$ einer einzelnen Schicht i des Hautbereichs abhängt.

4. Das Verfahren nach einem der Ansprüche 2 oder 3, wobei jeder charakterisierende Parameter pi die Permeabilität $\varepsilon_i$ einer einzelnen Schicht i des Hautbereichs ist.

5. Das Verfahren nach einem der Ansprüche 2 oder 3, wobei jeder charakterisierende Parameter pi der Wassergehalt einer einzelnen Schicht i des Hautbereichs ist.

6. Das Verfahren nach einem der vorangehenden Ansprüche, wobei die Messvorrichtung mindestens einen Satz von Elektroden mit einem gegenseitigen Abstand zwischen 0,1 mm und 0,2 mm umfasst.

7. Das Verfahren nach einem der vorangehenden Ansprüche, wobei die Messvorrichtung mindestens einen Satz von Elektroden mit einem gegenseitigen Abstand von mindestens 1 mm umfasst.

8. Das Verfahren nach einem der vorangehenden Ansprüche, wobei die angelegten elektrischen Felder eine Frequenz zwischen 50 MHz und 100 GHz aufweisen.

9. Das Verfahren nach einem der vorangehenden Ansprüche, umfassend die Schritte des wiederholten Bestimmens des mindestens einen charakterisierenden Parameters zu verschiedenen Zeiten nach dem Auftragen des nicht-therapeutischen Hautbehandlungsmittels.

10. Das Verfahren nach einem der vorangehenden Ansprüche, umfassend den Schritt des Messens einer optischen Reflexion oder Transmission des Hautbereichs.

11. Das Verfahren nach einem der vorangehenden Ansprüche, umfassend den Schritt des Messens einer Temperatur des Hautbereichs, und insbesondere, wobei ein Feuchtigkeitsgehalt einer Schicht i des Hautbereichs aus einer Permeabilität $\varepsilon_i$ der Schicht i und der Temperatur berechnet wird.

12. Das Verfahren nach einem der vorangehenden Ansprüche, umfassend den Schritt des Messens einer Leitfähigkeit des Hautbereichs für eine Frequenz unter 1 MHz.

**13.** Das Verfahren nach einem der vorangehenden Ansprüche, umfassend den Schritt des Messens der Umgebungsluftfeuchtigkeit.

**14.** Das Verfahren nach einem der vorangehenden Ansprüche, umfassend den Schritt des Messens einer Verdunstung aus dem Hautbereich.

**15.** Das Verfahren nach einem der vorangehenden Ansprüche, wobei die Sätze von Elektroden durch koplanare Wellenleiter gebildet werden.

**16.** Das Verfahren nach einem der vorangehenden Ansprüche, umfassend den Schritt des Bestimmens, aus den charakterisierenden Parametern, einer Entwicklung von Wasserkonzentrationen als Funktion der Zeit in mindestens einer Hautschicht nach dem Auftragen des nicht-therapeutischen Mittels.

**Revendications**

**1.** Un procédé pour caractériser un effet d'un agent non thérapeutique de traitement de la peau sur la peau, comprenant les étapes

(a) appliquer l'agent de traitement de la peau sur une région de peau,
(b) appliquer un dispositif de mesure à ladite région de peau, ledit dispositif de mesure comportant plusieurs ensembles d'électrodes (1, 2 ; 1a, 1b, 2), chaque ensemble comprenant au moins deux électrodes et les électrodes de chaque ensemble d'électrodes ayant une distance mutuelle Wi les unes des autres et dans lequel il existe N > 1 ensembles ayant différentes distances mutuelles Wi,
(c) générer, au moyen desdits différents ensembles desdites électrodes, au moins N champs électriques dans ladite région de peau, lesdits champs électriques ayant différentes profondeurs de pénétration dans ladite région de peau,
(d) mesurer au moins N paramètres mesurés mi, où chaque paramètre mi mesuré dépend d'une permittivité effective vue d'un différent desdits N champs électriques,
(e) calculer, à partir desdits paramètres mesurés mi, au moins un paramètre de caractérisation descriptif d'une permittivité de ladite région de peau pour une profondeur donnée,

ledit dispositif de mesure comprenant

- au moins un ensemble d'électrodes ayant une distance mutuelle inférieure ou égale à 0,1 mm et
- au moins un ensemble d'électrodes ayant une distance mutuelle d'au moins 0,1 mm.

**2.** Le procédé selon la revendication 1, dans lequel l'étape (e) comprend en outre
calculer, à partir desdits paramètres mesurés mi, de plusieurs paramètres de caractérisation pi, en particulier N paramètres de caractérisation pi, pour différentes profondeurs de ladite région de peau.

**3.** Le procédé selon la revendication 2, dans lequel chaque paramètre de caractérisation pi dépend d'une permittivité $\varepsilon_i$ d'une seule couche i de ladite région de peau.

**4.** Le procédé selon l'une quelconque des revendications 2 ou 3, dans lequel chaque paramètre de caractérisation pi est la permittivité $\varepsilon_i$ d'une seule couche i de ladite région de peau.

**5.** Le procédé selon l'une quelconque des revendications 2 ou 3, dans lequel chaque paramètre de caractérisation pi est la teneur en eau d'une seule couche i de ladite région de peau.

**6.** Le procédé selon l'une quelconque des revendications précédentes, dans lequel ledit dispositif de mesure comprend au moins un ensemble d'électrodes ayant une distance mutuelle comprise entre 0,1 mm et 0,2 mm.

**7.** Le procédé selon l'une quelconque des revendications précédentes, dans lequel ledit dispositif de mesure comprend au moins un ensemble d'électrodes ayant une distance mutuelle d'au moins 1 mm.

**8.** Le procédé selon l'une quelconque des revendications précédentes, dans lequel les champs électriques appliqués ont une fréquence comprise entre 50 MHz et 100 GHz.

**9.** Le procédé selon l'une quelconque des revendications précédentes, comprenant les étapes consistant à déterminer de manière répétitive, à différents moments après l'application de l'agent de traitement de la peau non thérapeutique, l'au moins un paramètre caractérisant.

**10.** Le procédé selon l'une quelconque des revendications précédentes, comprenant l'étape consistant à mesurer une réflexion ou une transmission optique de la région de la peau.

**11.** Le procédé selon l'une quelconque des revendications précédentes, comprenant l'étape consistant à mesurer une température de la région de peau, et en particulier une teneur en humidité d'une couche i de ladite région de peau étant calculée à partir d'une permittivité $\varepsilon_i$ de ladite couche i et de ladite température.

**12.** Le procédé selon l'une quelconque des revendications précédentes, comprenant l'étape consistant à mesurer une conductivité de ladite région de peau pour une fréquence inférieure à 1 MHz.

**13.** Le procédé selon l'une quelconque des revendications précédentes, comprenant l'étape consistant à mesurer l'humidité de l'air ambiant.

**14.** Le procédé selon l'une quelconque des revendications précédentes, comprenant l'étape consistant à mesurer une évaporation à partir de la région de la peau.

**15.** Le procédé selon l'une quelconque des revendications précédentes, dans lequel lesdits ensembles d'électrodes sont formés par des guides d'ondes coplanaires.

**16.** Le procédé selon l'une quelconque des revendications précédentes, comprenant l'étape consistant à déterminer, à partir desdits paramètres de caractérisation, une évolution des concentrations en eau en fonction du temps dans au moins une couche de peau après application de l'agent non thérapeutique.

**Fig. 1**

**Fig. 2**

**Fig. 3**

**Fig. 4**

**Fig. 5**

**Fig. 6**

**Fig. 7**

Fig. 8

Fig. 9

25

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6442408 B
- US 6966877 B
- JP 56118654 B
- WO 2004049937 A
- WO 2008154753 A
- WO 2005120332 A
- US 6070092 A
- US 20040240512 A

### Non-patent literature cited in the description

- **C.P. WEN.** Coplanar Waveguide: A Surface Strip Transmission Line Suitable for Nonreciprocal Gyromagnetic Device Applications. *IEEE Trans Microwave Theory Techn.,* December 1969, 1087-1090
- **S. GEVORGIAN ; L.J.P. LINNÉR ; E.L. KOLLBERG.** CAD Models for Shielded Multilayered CPW. *IEEE Trans Microwave Theory Techn.,* April 1995, 772-779
- **J. BAKER-JARVIS ; M.D. JANEZIC ; B.F. RIDDLE ; R.T. JOHNK ; P. KABOS ; CH.L. HOLLOWAY ; R.G. GEYER ; CH.A. GROSVENOR.** Measuring the Permittivity and Permeability of Lossy Materials: Solids, Liquids, Building Materials, and Negative-Index Materials. NIST Technical Note 1536, 2005
- **S.S. BEDAIR ; I. WOLFF.** Fast, Accurate and Simple Analytic Formulas for Calculating the Parameters of Supported Coplanar Waveguides for (M)MIC's. *IEEE Trans. Microwave Theory Techn,* January 1992, vol. 40, 41-48
- **M.D. JANEZIC ; D.F. WILLIAMS.** Permittivivty Characterization from Transmission-Line Measurements. *IEEE MTT-S Int Microwave Symposium Dig.,* 1343-1346
- **S.S. STUCHLY ; C.E. BASSEY.** Microwave coplanar sensors for dielectric measurements. *Meas Sci Technol.,* 1998, 1324-1329
- **A. RAJ ; W.S. HOLMES ; S.R. JUDAH.** Wide Bandwidth Measurement of Complex Permittivity of Liquids using Coplanar Lines. *IEE Trans. Instr. Meas.,* August 2001, vol. 50
- **B. KANG ; J. CHO ; CH. CHEON ; Y. KWON.** Nondestructive Measurements of Complex Permittivity and Permeability Using Multilayered Coplanar Waveguide Structures. *IEEE Microwave Wireless Comp. Lett.,* May 2005, vol. 15
- **C. VEYRES ; V.F. HANNA.** Extension of the application of conformal mapping techniques to coplanar lines with finite dimensions. *Int. J. Electron.,* 1980, vol. 48, 47-56
- **A. KRASZEWSKI ; S. KULINSKI ; M. MATUSZEWSKI.** Dielectric properties and a model of biphase water suspension at 9.4 GHz. *Journal of Applied Physics,* April 1976, vol. 47 (4), 1275-1277